# EUROPEAN PATENT APPLICATION

(11) **EP 2 236 509 A1**
(43) Date of publication of application: **06.10.2010**
(21) Application number: 09382043.9
(22) Date of filing: 01.04.2009
(51) Int. Cl.: C07D 405/06

(54) **Method for obtaining 1,3-difunctionalized pyrrolidine derivatives**

(71) Applicant: Ragactives, S.L., 47151 Boecillo (Valladolid) (ES)
(72) Inventor: Lorente Bonde-Larsen, Antonio, E-47151, Boecillo - Valladolid (ES); Rodriguez Lopez, María, E-47151, Boecillo - Valladolid (ES); Fernandez Sainz, Yolanda, E-47151, Boecillo - Valladolid (ES); Gutiérrez Fuentes, Luis Gerardo, E-47151, Boecillo - Valladolid (ES)
(74) Representative: ABG Patentes, S.L.

(57) **Abstract**

The present invention relates to a method for obtaining 1,3-difunctionalized pyrrolidine derivatives, such as 3-(S)-(-)-(1-carbamoyl-1,1-diphenylmethyl)-1-[2-(2,3-dihydrobenzofuran-5-yl)ethyl]pyrrolidine, commonly known as Darifenacin, as well as to the intermediates of said method and the respective methods for preparing them.

## Description

### Field of the Invention

The present invention relates to a method for obtaining 1,3-difunctionalized pyrrolidine derivatives, which include 3-(S)-(-)-(1-carbamoyl-1,1-diphenylmethyl)-1-[2-(2,3-dihydrobenzofuran-5-yl)ethyl]pyrrolidine, commonly known as Darifenacin.

### Background of the Invention

The 1,3-difunctionalized pyrrolidine derivatives referred to in this description are muscarinic receptor antagonists; specifically, Darifenacin is an active substance used in pharmaceutical preparations for the treatment of urinary incontinence.

The 1,3-difunctionalized pyrrolidine derivatives referred to in the invention, and more specifically Darifenacin, were first described in European patent EP 388054 B1, which describes several methods for obtaining them having a convergent route as a common nexus, as shown in Reaction Scheme 1, including as a key step a nucleophilic substitution reaction between a pyrrolidine derivative already functionalized in position 3 and a 5-benzofuranethyl or 5-(1,2-dihydrobenzofuranethyl) halide.

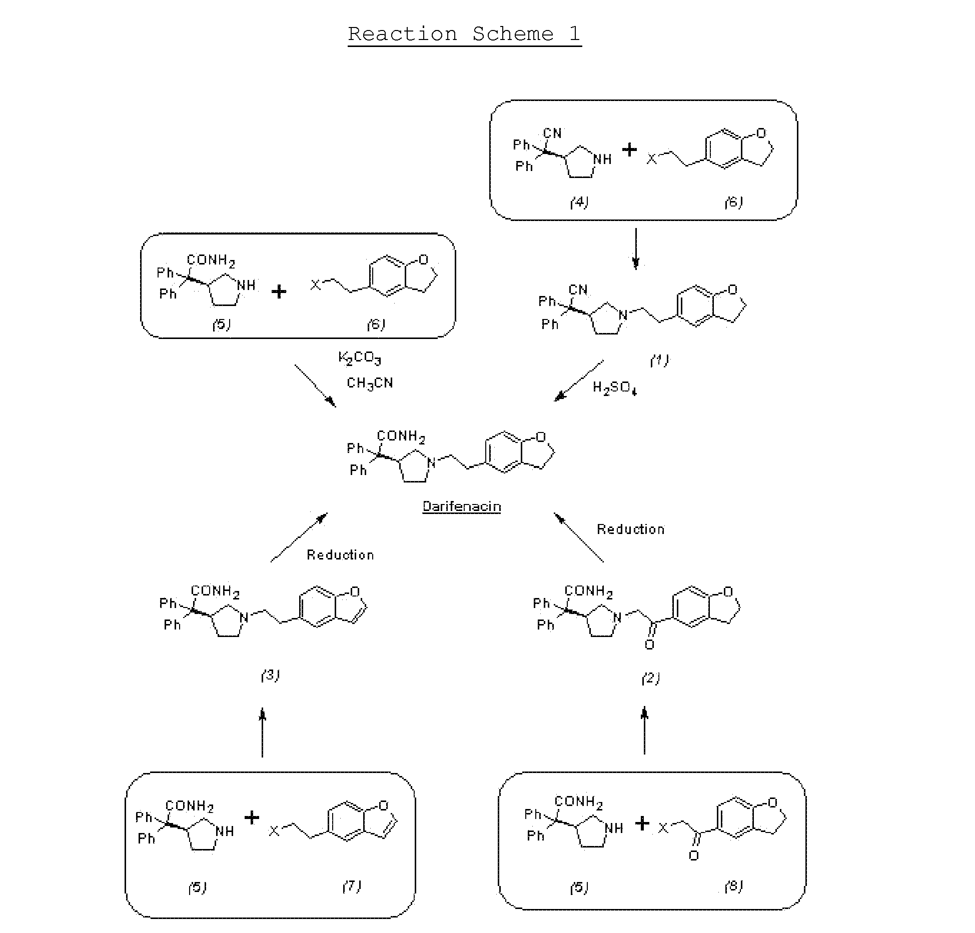

International patent application WO 2008/029257 A2 describes a method which improves the yield of said nucleophilic substitution, consisting of performing the reaction in the presence of a phase-transfer catalyst.

The limitation of these methods is the synthesis of the pyrrolidine derivative already functionalized in position 3 [compounds (4) or (5), according to Reaction Scheme 1] (Chem. Lett. 1966, 893 and EP 178947), since it consists of 5 or 7 reaction steps, respectively, with an overall molar yield of 3.57% or 0.54%, respectively. As shown in Reaction Scheme 2, the low yield of said methods seems to be associated to two main facts: on one hand, the need to reverse the configuration of the carbon supporting the hydroxy group in position 3 (Mitsunobu reaction), which involves the need for a second purification step by means of diastereomeric salt resolution, probably due to a partial epimerization of said stereogenic center; and on the other hand, to the need of protecting the pyrrolidine in the form of sulfonamide to carry out the O-tosylation and substitution reaction with diphenylacetonitrile, which involves two additional synthesis steps.

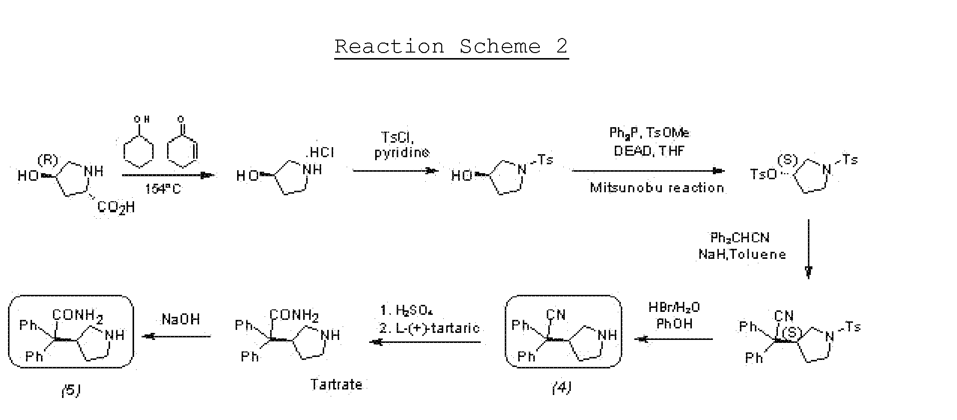

The importance of protecting the nitrogen of 3-hydroxypyrrolidine before carrying out the O-tosylation reaction is clearly shown in United States patent application US2008/0214835 A1, which describes a series of protecting groups which include sulfonamides, amides, alkyl/aryl silylamines or alkyl/aryl thio amines.

United States patent US 6930188 B2 describes the formation of a Darifenacin solvate with toluene, basically using the same strategy defined in European patent EP 388054, with the difference that the convergent step consists of the reaction between 2-(1,2-dihydrobenzofuran-5-yl)acetic acid and 3-(S)-(1-cyano-1,1-diphenylmethyl)pyrrolidine (Reaction Scheme 3).

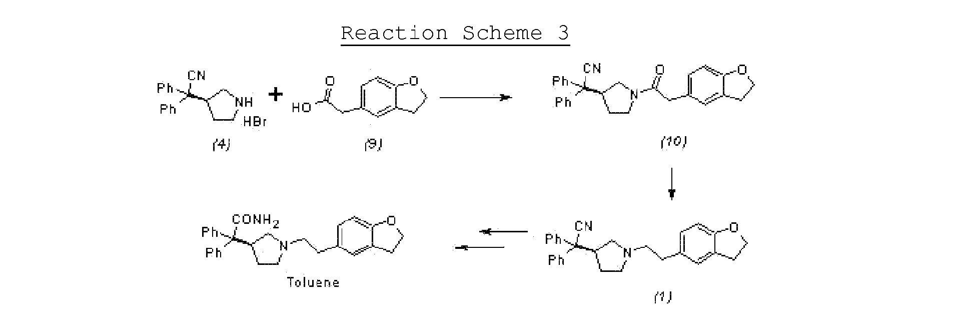

Likewise, United States patent US 7442806 B2 describes obtaining Darifenacin by means of the formation of an enamine and the subsequent reduction thereof (Reaction Scheme 4).

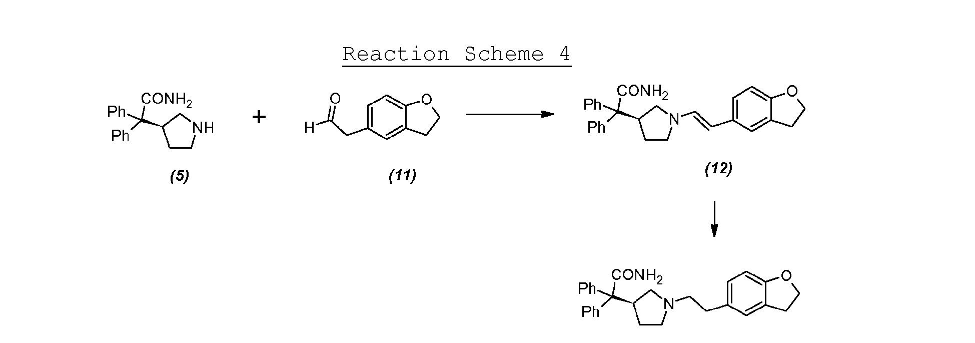

International patent application WO 2008/000418 A2 makes use of the same strategy described in EP 388054 (and therefore has the same limitations), with the exception that other pyrrolidine derivatives already functionalized in position 3 are used, specifically, 3-(S)-(1-carboxyalkyl-1,1-diphenylmethyl)pyrrolidine or 3-(S)-(1-carbamoylderivative-1,1-diphenylmethyl) pyrrolidine, which also subsequently give rise to Darifenacin (Reaction Scheme 5).

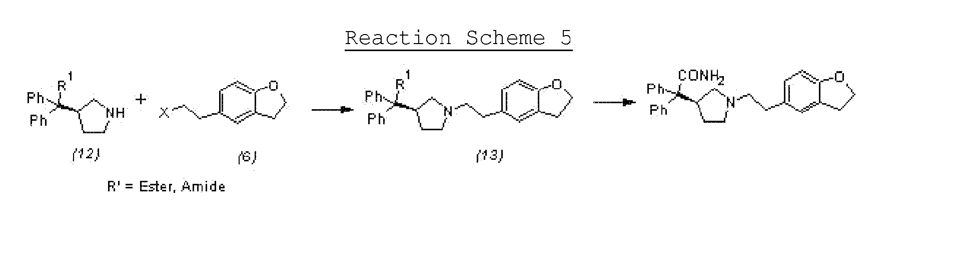

Therefore, there is a need to develop an alternative method for obtaining 1,3-difunctionalized pyrrolidine derivatives which overcomes all or part of the problems associated with the known methods belonging to the state of the art. Advantageously, said alternative method must start from a commercially available starting material, it must take place in few synthesis steps, thus favoring the increased yield and it must prevent reaction conditions preventing its industrialization.

### Summary of the Invention

The present invention faces the problem of providing an alternative method for obtaining 1,3-difunctionalized pyrrolidine derivatives of general formula (I) (defined below), solvates, enantiomers or pharmaceutically acceptable salts thereof, which include Darifenacin, which overcomes the problems existing in the different syntheses of the state of the art mentioned above.

The solution provided by the invention is based on the fact that the inventors have observed that it is surprisingly possible to efficiently obtain 3-hydroxypyrrolidine derivatives functionalized in position 1 of general formula (IV) (defined below) by means of a method [see Reaction Scheme 6] comprising firstly forming a cyclic imide by reacting an amine of general formula (VI) (defined below) with malic acid [compound of formula (VII)], whereby obtaining the corresponding derivative of 1-functionalized 3-hydroxypyrrolidin-2,5-dione of general formula (V) (defined below), and secondly reducing the imide group by means of the use of a reducing agent. Subsequently, the 3-hydroxyl group of said 1-functionalized 3-hydroxypyrrolidine derivative of general formula (IV) is transformed into a good leaving group, whereby obtaining a compound of general formula (II) (defined below) which, after reacting with a compound of general formula (III) (defined below), gives rise to a 1,3-difunctionalized pyrrolidine derivative of general formula (I), solvates, enantiomers or pharmaceutically acceptable salts thereof, including Darifenacin. The previously indicated transformations are shown in Reaction Scheme 6. 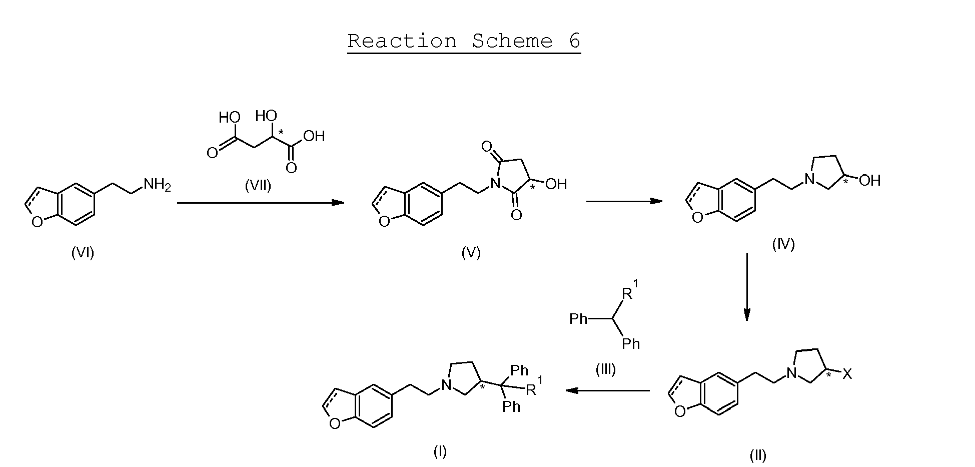 wherein
the dotted line is absent or represents a bond between the carbon atoms bound by said dotted line;
* represents an asymmetric carbon;
Ph represents a phenyl group;
R¹ is CN, CONR²R³ or CO₂R², wherein R² and R³ represent independently of one another H, C₁-C₅ alkyl, or benzyl; and X is a leaving group.

As can be seen in said Reaction Scheme 6, the compounds of general formula (IV) form intermediates useful for obtaining 1,3-difunctionalized pyrrolidine derivatives of general formula (I), solvates, enantiomers or pharmaceutically acceptable salts thereof.

The method provided by the present invention for obtaining the 1,3-difunctionalized pyrrolidine derivatives of general formula (I) have a number of advantages since, on one hand, it allows the use of readily available starting materials, and on the other hand allows substantially reducing the synthesis steps necessary for obtaining said compounds of general formula (I). Functionalizing the pyrrolidine in position 1 in the first synthesis steps prevents the need to protect/deprotect the amino group. Furthermore, the use of optically pure malic acid allows accessing pyrrolidines with a determined spatial configuration in position 3, preventing the need for a subsequent diastereomeric resolution. Particularly, L-(-)-malic acid is useful for obtaining 3-(S)-1,3-difunctionalized pyrrolidines and D-(+)-malic acid is useful for obtaining 3-(R)-1,3-difunctionalized pyrrolidines. Finally, and surprisingly, unlike what happens with the pyrrolidines protected as sulfonamides described in the state of the art, the introduction of the diphenylacetonitrile group is achieved by means of reversing the configuration of the chiral center, in a completely stereoselective manner, whereby it is not necessary to perform a subsequent resolution of the compound, with the subsequent saving in steps and a higher yield. This all contributes to obtaining an industrial process with few synthesis steps.

Therefore, in one aspect, the invention relates to a method for obtaining a compound of general formula (I), an enantiomer, a solvate or a pharmaceutically acceptable salt thereof, comprising firstly transforming the hydroxyl group in position 3 of a compound of general formula (IV) into a leaving group to give rise to a compound of general formula (II); and secondly reacting said compound of general formula (II) with a compound of general formula (III), wherein in the case of compounds with a defined configuration in position 3 of the pyrrolidine ring, a reversal of the configuration is achieved in a completely stereoselective manner. If desired, a compound of general formula (I) can be converted into another compound of general formula (I). In a preferred embodiment, the compound of general formula (I) obtained is 3-(S)-(-)-(1-carbamoyl-1,1-diphenylmethyl)-1-[2-(2,3-dihydrobenzofuran-5-yl)ethyl]pyrrolidine (Darifenacin), a solvate or a pharmaceutically acceptable salt thereof.

In another aspect, the invention relates to a compound of general formula (II) selected from 1-[2-(benzofuran-5-yl)ethyl]-3-paratoluenesulfonyloxy pyrrolidine, 1-[2-(benzofuran-5-yl)ethyl]-3-methylsulfonyloxy pyrrolidine, 1-[2-(benzofuran-5-yl)ethyl]-3-trifluoromethylsulfonyloxy pyrrolidine, 1-[2-(benzofuran-5-yl)ethyl]-3-chloro pyrrolidine, 1-[2-(2,3-dihydrobenzofuran-5-yl)ethyl]-3-paratoluenesulfonyloxy pyrrolidine, 1-[2-(2,3-dihydrobenzofuran-5-yl)ethyl]-3-methylsulfonyloxy pyrrolidine, 1-[2-(2,3-dihydrobenzofuran-5-yl)ethyl]-3-trifluoromethylsulfonyloxy pyrrolidine, 1-[2-(2,3-dihydrobenzofuran-5-yl)ethyl]-3-chloro pyrrolidine, enantiomers, solvates or salts thereof.

In another aspect, the invention relates to a compound of general formula (IV) selected from 1-[2-(2,3-dihydrobenzofuran-5-yl)ethyl]-3-hydroxypyrrolidine and 1-[2-[(benzofuran-5-yl)ethyl]-3-hydroxypyrrolidine, enantiomers, solvates or salts thereof.

In another aspect, the invention relates to a method for obtaining a compound of general formula (IV), an enantiomer, a solvate or a salt thereof, comprising the reduction of a compound of general formula (V) by means of the use of a reducing agent.

In another aspect, the invention relates to a compound of general formula (V) selected from 3-hydroxy-1-[2-(2,3-dihydrobenzofuran-5-yl)ethyl]-pyrrolidin-2,5-dione and 3-hydroxy-1-[2-(benzofuran-5-yl)ethyl]-pyrrolidin-2,5-dione, enantiomers, solvates or salts thereof.

In another aspect, the invention relates to a method for obtaining a compound of general formula (V), an enantiomer, a solvate or a salt thereof, by means of an imidation reaction of the malic acid of formula (VII) and of a compound of general formula (VI).

### Detailed Description of the Invention

### Definitions

As it is used herein, the term "C₁-C₅ alkyl" relates to a radical derived from a linear or branched alkane, with 1 to 5 carbon atoms, for example, methyl, ethyl, propyl, n-butyl, tert-butyl, etc.

As it is used herein, the term "leaving group" includes any group capable of being detached from the pyrrolidine present in the compound of general formula (II) together with its binding electrons during the reaction with the functionalized diphenylmethylene derivative of general formula (III). Illustrative examples of said leaving groups can be found in general organic chemistry and advanced organic chemistry overviews, such as March's Advanced Organic Chemistry: Reactions, Mechanisms, and Structure. Sixth Edition By Michael B. Smith and Jerry March. John Wiley & Sons, Inc., Hoboken, NJ. 2007. ISBN 978-0-471-72091-1. Virtually any leaving group can be used for putting the invention into practice; nevertheless, the leaving group is preferably selected from an (C₁-C₄)alkylsulfonyloxy, arylsulfonyloxy, trifluoromethanesulfonyloxy or halide group.

The invention also provides "salts" of the compounds described in the present description. By way of illustration, said salts can be acid addition salts, base addition salts or metal salts, and they can be synthesized from the parent compounds containing a basic or acid residue by means of conventional chemical methods known by persons skilled in the art. Such salts are generally prepared, for example, by reacting the free base or acid forms of said compounds with a stoichiometric amount of the suitable base or acid in water or in an organic solvent or in a mixture of both. Non-aqueous media such as ether, ethyl acetate, ethanol, acetone, isopropanol or acetonitrile, are generally preferred. Illustrative examples of said acid addition salts include inorganic acid addition salts such as, for example, hydrochloride, hydrobromide, hydroiodide, sulfate, nitrate, phosphate, etc., organic acid addition salts such as, for example, acetate, maleate, fumarate, citrate, oxalate, succinate, tartrate, malate, mandelate, methanesulfonate, p-toluenesulfonate, etc. Illustrative examples of base addition salts include salts of inorganic bases such as, for example, ammonium salts and salts of organic bases such as, for example, ethylenediamine, ethanolamine, N,N-dialkylenethanolamine, triethanolamine, glutamine, amino acid basic salts, etc. Illustrative examples of metal salts include, for example, sodium, potassium, calcium, magnesium, aluminum and lithium salts.

Likewise, the compounds described in the present description can be obtained in free acid or base form or, alternatively, in the form of salt. In both cases they can be obtained in crystalline form, both as free compounds or as solvates (e.g., hydrates, alcoholates, etc.), both forms being included within the scope of the present invention. The solvation methods are generally known in the state of the art.

A "stereoisomer" is understood for the person skilled in the art as compounds formed by the same atoms bound by the same sequence of bonds, but with different three-dimensional structures which are not interchangeable. The individual stereoisomers, both enantiomers and diastereoisomers, as well as mixtures thereof, fall within the scope of the present invention. Said individual enantiomers or diastereoisomers, as well as mixtures thereof, can be separated by means of conventional techniques. Specifically, it is evident for the person skilled in the art that position 3 of the pyrrolidine ring, in the compounds of general formulas (V), (IV), (II) and (I), is an asymmetric center and that it can therefore have spatial configuration (R) or (S). Even though the invention can take place using mixtures of enantiomers of said compounds of general formulas (V), (IV), (II) and (I), e.g., racemic mixtures, in practice the use of single enantiomers of said compounds is preferred. In this sense, when putting the present invention into practice, it is advantageous to use optically pure malic acid in the synthesis of a compound of general formula (V) for the purpose of obtaining a single enantiomer. Nevertheless, diastereomeric resolutions for example could also be carried out in each of the compounds of general formulas (IV), (II) and (I) to obtain a single enantiomer.

The term "pharmaceutically acceptable" relates to the fact that the molecular entities and compositions are physiologically tolerable and usually do not cause an allergic or similar adverse reaction, such as gastric discomfort, dizziness and the like, when they are administered to a human being. Preferably, as it is used in this description, the term "pharmaceutically acceptable" means approved by a regulatory agency of the state or federal government, or listed in the US pharmacopoeia or other generally recognized pharmacopoeia for its use in animals, and more particularly in humans.

For those persons skilled in the art, it will be evident that the scope of the present invention also includes salts which are not pharmaceutically acceptable as possible means for obtaining pharmaceutically acceptable salts.

Unless otherwise indicated, the compounds of the invention also include compounds which differ only in the presence of one or more isotopically enriched atoms. By way of illustration, compounds having said structure, with the exception of the substitution of a hydrogen with a deuterium or with tritium, or the substitution of a carbon with a carbon enriched with ¹³C or ¹⁴C or a nitrogen enriched with ¹⁵N, are within the scope of this invention.

### Obtaining a Compound of General Formula (V)

In one aspect, the invention relates to a method for obtaining a compound of general formula (V) 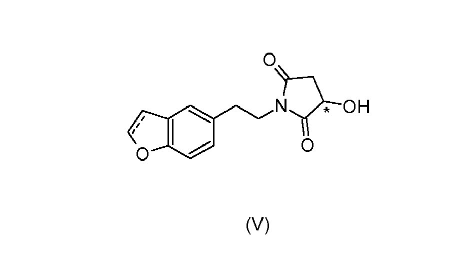 wherein
the dotted line is absent or represents a bond between the carbon atoms bound by said dotted line; and
* represents an asymmetric carbon,
or an enantiomer, a solvate or a salt thereof,
comprising reacting a compound of general formula (VI) 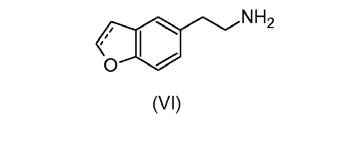 wherein the dotted line has the previously mentioned meaning, with an acid of formula (VII) 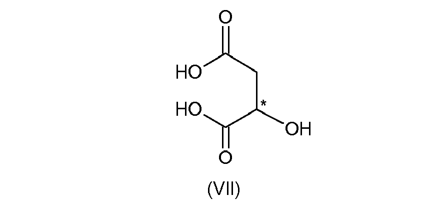 wherein the asterisk (*) has the previously mentioned meaning.

According to the present invention, the compound of general formula (V) can be obtained by means of a method such as the one shown in Reaction Scheme 7. 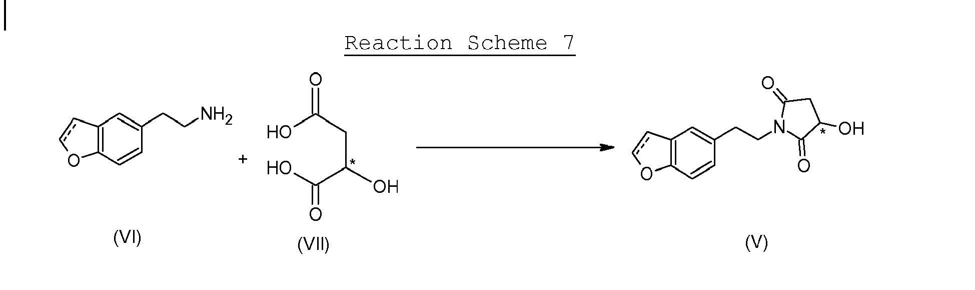 wherein the dotted line and the asterisk (*) have the previously mentioned meanings.

The reaction of the amine of formula (VI) with the acid of formula (VII) can be carried out in a suitable solvent, such as an organic solvent, for example, an aromatic solvent (e.g., toluene, xylene, etc.), an alcohol, (e.g., ethanol, etc.), a halogenated solvent (e.g., chloroform, etc.), an amide (e.g., dimethylformamide), etc. In a particular embodiment, said organic solvent is selected from the group consisting of toluene, xylene, ethanol, chloroform, dimethylformamide and mixtures thereof; preferably, xylene.

Preferably, the imidation reaction is carried out under heating, for example, under reflux conditions of the solvent used, in which the elimination of the water formed from the reaction medium is favored, whereby favoring the formation of the imide group; under these conditions, the reaction can be completed in a time period comprised between approximately 1 and 8 hours.

Depending on the desired stoichiometric, either configuration of the acid of formula (VII) will be used. Thus, the use of L-(-)-malic acid allows obtaining a 1,3-difunctionalized 3-(S)-pyrrolidine and the use of D-(+)-malic acid allows obtaining a 1,3-difunctionalized 3-(R)-pyrrolidine. Nevertheless, if desired, it is also possible to use malic acid which is not optically pure, e.g., a mixture of malic acid enantiomers in the form of a racemic mixture or in the form of a mixture enriched with one of the enantiomers. In a particular and preferred embodiment, the compound of formula (VII) is L-(-)-malic acid.

The amine (VI) can be obtained from a commercial acid (IX) in two steps (amidation and reduction) as shown in Reaction Scheme 8. The conditions of these transformations are known. 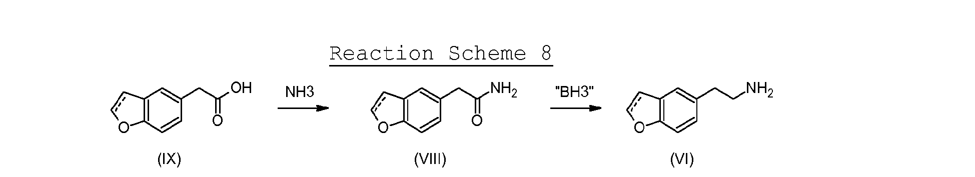 wherein the dotted line has the previously mentioned meaning.

The compound of formula (VII), malic acid, enantiomers thereof, L- (-) -malic acid and D-(+)-malic acid, and mixtures thereof, are commercially available compounds.

The obtained compounds of formula (V) can be used directly in the next reaction or they can be purified, by means of conventional and industrially acceptable processes, such as, for example, by means of a crystallization process. Illustrative, non-limiting examples of suitable solvents for said crystallization include an organic solvent, for example, a ketone (e.g., acetone, etc.), a nitrile (e.g., acetonitrile, etc.), an alcohol (e.g., isopropanol (i-PrOH), ethanol (EtOH), etc.), optionally mixed with water. In a preferred embodiment, said solvent is selected from EtOH, i-PrOH and mixtures thereof with water.

In a particular embodiment, the obtained compound of formula (V) is a compound in which the dotted line is absent, whereas in another particular embodiment, the obtained compound of formula (V) is a compound in which the dotted line represents a bond between the carbon atoms bound by said dotted line. Illustrative examples of compounds of general formula (V) include 3-hydroxy-1-[2-(2,3-dihydrobenzofuran-5-yl)ethyl]-pyrrolidin-2,5-dione (Va), 3-hydroxy-1-[2-(benzo-furan-5-yl)ethyl]-pyrrolidin-2,5-dione (Vb) 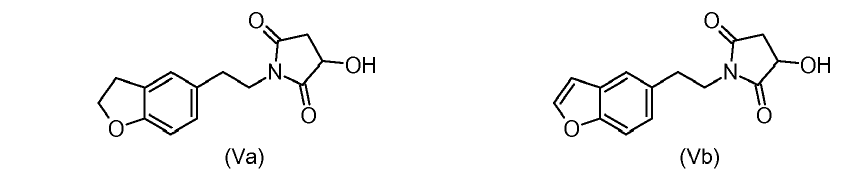 enantiomers, solvates or salts thereof.

In a preferred embodiment, the configuration in position 3 of the pyrrolidine is (S), giving rise to 3-(S)-hydroxy-1-[2-(2,3-dihydrobenzofuran-5-yl)ethyl]-pyrrolidin-2,5-dione, or 3-(S)-hydroxy-1-[2-(benzofuran-5-yl)ethyl]-pyrrolidin-2,5-dione,
or a solvate or a salt thereof.

The compounds of formula (V) are an additional aspect of this invention.

Obtaining a Compound of General Formula (IV)

In another aspect, the invention relates to a method for obtaining a compound of general formula (IV) 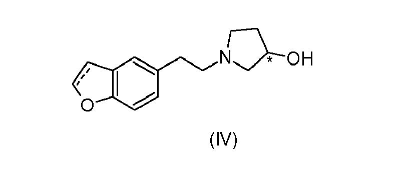 wherein
the dotted line is absent or represents a bond between the carbon atoms bound by said dotted line; and
* represents an asymmetric carbon,
or an enantiomer, a solvate or a salt thereof,
comprising reacting a compound of general formula (V) 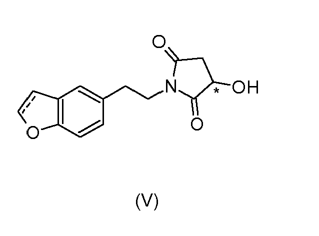 wherein the dotted line and the asterisk (*) have the previously mentioned meanings,
or an enantiomer, solvate or salt thereof,
with a reducing agent.

Therefore, according to the invention, the compound of general formula (IV) can be obtained by means of a method such as the one shown in Reaction Scheme 9. 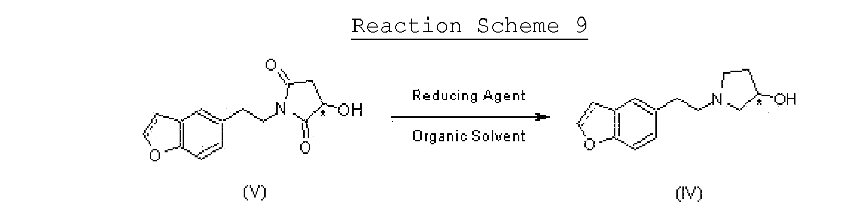 wherein the dotted line and the asterisk (*) have the previously mentioned meanings. The compound of formula (V), enantiomers, solvates or salts thereof, can be obtained by means of a method such as the one previously described and shown in Reaction Scheme 7 by means of the reaction of an amine of formula (VI) with an acid of formula (VII).

Illustrative, non-limiting examples of said reducing agent used in this reaction include a hydride, e.g., lithium aluminum hydride (LiAlH₄), sodium bis(2-methoxyethoxy)aluminum hydride (REDAL), aluminum hydride (AlH₃), etc.; diborane (B₂H₆), etc., and mixtures thereof. The diborane can be a diborane generated in the medium by means of, for example, I₂/sodium borohydride (NaBH₄), BF₃OEt₂/NaBH₄, by means of reacting NaBH₄ with BF₃ adducts with di-n-butylether, tert-butylether, monoglyme, dioxane or tetrahydropyran (Inorg. Chem., 2000, 39(8):1795-1802), etc.; or a commercial diborane in solution such as, for example, BH₃/Me₂S, etc. Lithium aluminum hydride (LiAlH₄) is the preferred reducing agent.

This reduction reaction is preferably carried out in a reaction medium comprising a solvent, such as an organic solvent, for example, an ether type medium (e.g., tetrahydrofuran (THF), 2-methyltetrahydrofuran, 1,2-dimethoxyethane, etc.), optionally mixed with another organic solvent, e.g., toluene, etc.; in a particular embodiment, the reduction of the compound of formula (V) is carried out with a reducing agent in a reaction medium comprising THF or a mixture of THF and toluene.

The reduction reaction is suitably carried out under heating, such as for example under reflux conditions of the solvent used. In these conditions, the reaction can be completed in a time period comprised between approximately 1 and 8 hours.

The obtained products of formula (IV) generally do not require subsequent purification but, if desired, they can additionally be purified by means of conventional and industrially acceptable processes, such as for example by means of a crystallization process, either of the product as a free base or through the formation of an addition salt. Illustrative, non-limiting examples of suitable solvents for said crystallization include a ketone (e.g., acetone, etc.), a nitrile (e.g., acetonitrile, etc.), an alcohol (e.g., i-PrOH, EtOH, etc.), an ester (e.g., ethyl acetate (AcOEt), etc.), an ether, etc., preferably acetone or acetonitrile.

In a particular embodiment, the obtained compound of formula (IV) is a compound in which the dotted line is absent, whereas in another particular embodiment, the obtained compound of formula (IV) is a compound in which the dotted line represents a bond between the carbon atoms bound by said dotted line. Illustrative examples of compounds of general formula (IV) include 1-[2-(2,3-dihydrobenzofuran-5-yl)ethyl]-3-hydroxypyrrolidine (IVa), 1-[2-(benzofuran-5-yl)ethyl]-3-hydroxypyrrolidine (IVb) 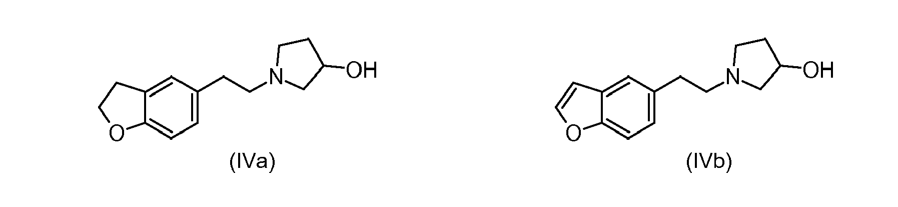 enantiomers, solvates or salts thereof.

In a preferred embodiment, the configuration in position 3 of the pyrrolidine is (S), giving rise to 3-(S)-1-[2-(2,3-dihydrobenzofuran-5-yl)ethyl]-3-hydroxy-pyrrolidine, or 3-(S)-1-[2-(benzofuran-5-yl)ethyl]-3-hydroxy-pyrrolidine, solvates or salts thereof.

The compounds of formula (IV) are an additional aspect of this invention.

### Obtaining a Compound of General Formula (II)

The invention also provides a method for obtaining a compound of general formula (II) comprising the conversion of the hydroxyl bound to the carbon in position 3 of the pyrrolidine present in a compound of general formula (IV), an enantiomer, solvate or salt thereof, into a leaving group to obtain a compound of general formula (II), an enantiomer, solvate or salt thereof, as shown in Reaction Scheme 10. 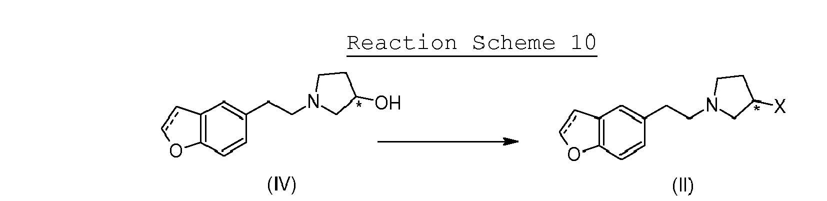 wherein
the dotted line is absent or represents a bond between the carbon atoms bound by said dotted line;
* represents an asymmetric carbon; and
X represents a leaving group.

Though virtually any leaving group can be present in the compound of general formula (II), in a particular embodiment, X is a ( C₁-C₄)alkylsulfonyloxy, arylsulfonyloxy, trifluoromethanesulfonyloxy or halide group.

Therefore, in a particular embodiment, said conversion reaction for converting the hydroxyl into a leaving group is carried out by treatment with a sulfonylating agent or with a halogenating agent to obtain a compound of general formula (II), an enantiomer, solvate or salt thereof, in which X is a leaving group such as a (C₁-C₄)alkylsulfonyloxy, arylsulfonyloxy, trifluoromethanesulfonyloxy or halide group.

Though virtually any sulfonylating agent can be used, in a particular preferred embodiment, the sulfonylating agent is p-toluenesulfonyl chloride. When said sulfonylating agent is used, the reaction is performed in the presence of an organic base, such as diisopropylethylamine, ethylenediamine, pyridine, triethylamine etc., preferably triethylamine or diisopropylethylamine, and optionally in the presence of a catalyst, for example, dimethylaminopyridine (DMAP), etc., in a reaction medium comprising a suitable solvent, such as an organic halogenated solvent (e.g., dichloromethane, 1,2 dichloroethane, etc.), an ester-type organic solvent (e.g., ethyl acetate, n-butyl acetate, isopropyl acetate, etc.), an ether-type organic solvent (e.g., THF, 2-methyl-THF, etc.), an aromatic solvent (e.g., toluene, xylene, etc.), a polar aprotic solvent (e.g., acetonitrile, acetone, methylisobutylketone, etc.), a dipolar aprotic solvent (e.g., dimethylformamide (DMF), dimethylsulfoxide (DMSO), etc.), etc.

The sulfonylation reaction is preferably performed in the presence of triethylamine or diisopropylethylamine as base, dichloromethane as solvent and DMAP as catalyst, at reflux temperature; under these conditions the reaction can be completed during a time period comprised between approximately 2 and 8 hours.

The obtained products of formula (II) generally do not require subsequent purification but if desired, they can additionally be purified by means of conventional and industrially acceptable processes such as, for example, by means of a crystallization process, either of the product as a free base (in the event that it is a solid) or, more preferably, through the formation of an addition salt (in the event that it is an oil). Illustrative, non-limiting examples of suitable solvents for said crystallization include ketones (e.g., acetone, etc.), nitriles (e.g., acetonitrile, etc.), alcohols (e.g., i-PrOH, EtOH, etc.), ethers, etc., preferably acetonitrile or acetone.

In a particular embodiment, the compound of formula (II) is a compound in which the dotted line is absent, whereas in another particular embodiment, the compound of formula (II) is a compound in which the dotted line represents a bond between the carbon atoms bound by said dotted line. Illustrative, non-limiting examples of compounds of general formula (II) include 1-[2-(benzofuran-5-yl)ethyl]-3-*p*-toluenesulfonyl-pyrrolidine, 1-[2-(benzofuran-5-yl)ethyl]-3-methyl-sulfonyl-pyrrolidine, 1-[2-(benzofuran-5-yl)ethyl]-3-trifluoromethylsulfonyl-pyrrolidine, 1-[2-(benzofuran-5-yl)ethyl]-3-chloro-pyrrolidine, 1-[2-(2,3-dihydrobenzofuran-5-yl)ethyl]-3-*p*-toluene-sulfonyl-pyrrolidine, 1-[2-(2,3-dihydrobenzofuran-5-yl)ethyl]-3-methylsulfonyl-pyrrolidine, 1-[2-(2,3-dihydrobenzofuran-5-yl)ethyl]-3-trifluoromethylsulfonyl-pyrrolidine, 1-[2-(2,3-dihydrobenzofuran-5-yl)ethyl]-3-chloro pyrrolidine, enantiomers, solvates or salts thereof; preferably, the compounds 1-[2-(2,3-dihydrobenzofuran-5-yl)ethyl]-3-*p-*toluene-sulfonyl-pyrrolidine
or 1-[2-(benzofuran-5-yl)ethyl]-3-*p*-toluenesulfonyl-pyrrolidine, enantiomers, solvates or salts thereof, or the compounds 3-(S)-1-[2-(2,3-dihydrobenzofuran-5-yl)ethyl]-3-*p*-toluenesulfonylpyrrolidine or 3-(S)-1-[2-(benzofuran-5-yl)ethyl]-3-*p-*toluenesulfonyl-pyrrolidine, solvates or salts thereof.

### Obtaining a Compound of General Formula (I)

The compound of general formula (II), enantiomers, solvates or salts thereof, can be used in the production of a compound of general formula (I), enantiomers, solvates or salts thereof.

Therefore, the invention provides a method, hereinafter Method A, for obtaining a compound of general formula (I) 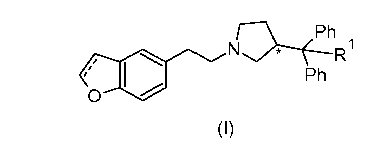 wherein
the dotted line is absent or represents a bond between the carbon atoms bound by said dotted line;
* represents an asymmetric carbon;
Ph represents a phenyl group; and
R¹ is CN, CONR²R³ or CO₂R², wherein R² and R³, represent
independently of one another H, C₁-C₅ alkyl, or benzyl,
or an enantiomer, a solvate or a pharmaceutically acceptable salt thereof, comprising reacting a compound of general formula (II) 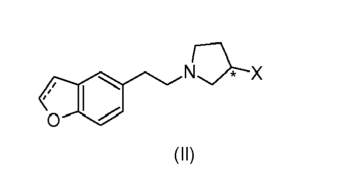 wherein
the dotted line and the asterisk (*) have the previously mentioned meanings; and
X is a leaving group;
an enantiomer, a solvate, or a salt thereof, with a compound of general formula (III), 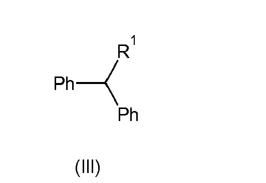 wherein Ph and R¹ have the previously mentioned meanings,
to obtain a compound of general formula (I), and, if desired, converting the compound of general formula (I) into another compound of general formula (I), an enantiomer, a solvate or a salt thereof, as shown in Reaction Scheme 11. 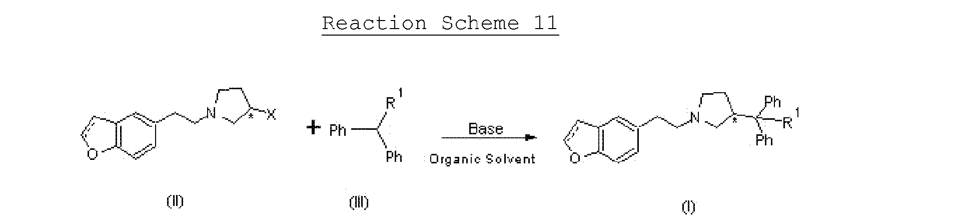 wherein the dotted line, the asterisk (*), Ph, R¹ and X have the previously mentioned meanings.

The compound of general formula (II) can be obtained by means of a method such as the one described above. In a particular embodiment, X is a (C₁-C₄)alkylsulfonyloxy, arylsulfonyloxy, trifluoromethanesulfonyloxy or halide group.

The reaction of the compound of formula (II) with the compound of formula (III) can be carried out in the presence of a base in a reaction medium comprising a suitable solvent.

Though virtually any base can be used in said reaction, in a particular embodiment, the base used is an inorganic base, such as a metal hydroxide (e.g., NaOH, KOH, etc.), a metal hydride (e.g., NaH, etc.), etc., or an organic base, such as a base of an alkoxide (e.g., potassium tert-butoxide (t-BuOK), sodium tert-butoxide (t-BuONa), potassium methoxide (KOMe), sodium methoxide (NaOMe), etc.), a metal amide, such as a metal amide of formula M-NH₂, wherein M is the cation of an alkali metal (e.g., LiNH₂, NaNH₂, KNH₂, etc.), etc. In a particular embodiment, said base is selected from the group consisting of NaOH, KOH, NaH, t-BuOK, t-BuONa, KOMe, NaOMe, LiNH₂, NaNH₂, KNH₂ and mixtures thereof, preferably, LiNH₂.

Suitable solvents for carrying out this reaction include aromatic organic solvents (e.g., toluene, xylene, etc.), ethers (methyl tert-butyl ether (MTBE), 2-methyltetrahydrofuran, etc.), dimethylsulfoxide, dimethylformamide (DMF), etc. and mixtures thereof. In a particular preferred embodiment, the organic solvent is a mixture of toluene and DMF.

The substitution reaction is suitably carried out under heating, for example at temperatures comprised between 50°C and the reflux temperature of the solvent used. Under these conditions, the reaction can be completed in a time period comprised between approximately 2 and 12 hours.

A compound of general formula (I) can optionally be converted into another compound of general formula (I) by conventional methods known by persons skilled in the art; by way of non-limiting illustration:
- a compound of general formula (I) wherein R¹ is a nitrile group can be converted into another compound of general formula (I) by means of hydrolysis;
- a compound of general formula (I) wherein R¹ is a protected amido group can be converted into another compound of general formula (I) by means of a deprotection reaction; or
- a compound of general formula (I) wherein R¹ is an ester group can be converted into another compound of general formula (I) by means of an amidation reaction or by means of a hydrolysis reaction.

In a particular preferred embodiment, a compound of general formula (I) wherein R¹ is a nitrile group [(I), R¹ = CN] can be converted into another compound of general formula (I) either by means of hydrolysis in acid medium (e.g., using sulfuric acid in the absence of solvent at a temperature comprised between 50°C and 90°C) or by means of hydrolysis in basic medium (e.g., using 2-methyl-2-butanol in the presence of KOH) following methods already described in the literature, such as those mentioned in EP 388054 (hydrolysis in acid medium) or US 6930188 (hydrolysis in basic medium), to give rise to the carbamoyl group in formula (I), wherein the compound 3-(S)-(-)-(1-carbamoyl-1,1-diphenylmethyl)-1-[2-(2,3-dihydrobenzofuran-5-yl)ethyl]
pyrrolidine, corresponds to Darifenacin.

Alternatively, in another aspect the invention relates to a method, hereinafter Method B, for obtaining a compound of general formula (I) 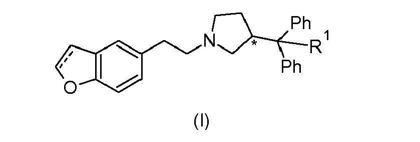 wherein
the dotted line is absent or represents a bond between the carbon atoms bound by said dotted line;
* represents an asymmetric carbon;
Ph represents a phenyl group; and
R¹ is CN, CONR²R³ or CO₂R², wherein R² and R³, represent independently of one another H, C₁-C₅ alkyl, or benzyl,
or an enantiomer, a solvate or a pharmaceutically acceptable salt thereof, comprising:
a) transforming the hydroxyl group bound to the carbon in position 3 of the pyrrolidine present in a compound of general formula (IV), a solvate, an enantiomer or a salt thereof, 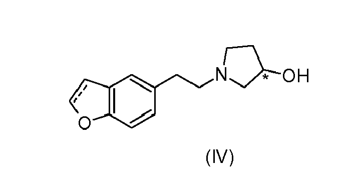 wherein the dotted line and the asterisk (*) have the previously mentioned meanings,
   into a leaving group to obtain a compound of general formula (II), a solvate, an enantiomer or a salt thereof, 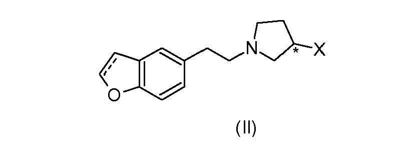 wherein the dotted line and the asterisk (*) have the previously mentioned meanings, and
   X is a leaving group;
b) reacting said compound of general formula (II), enantiomer, solvate or salt thereof, with a compound of general formula (III), 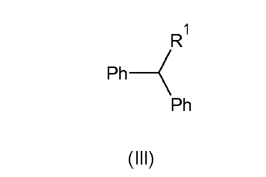 wherein Ph and R¹ have the previously mentioned meanings,
   to obtain a compound of general formula (I), and, if desired,
c) converting the compound of general formula (I), an enantiomer, a solvate or a salt thereof, into another
   compound of general formula (I).

Step a) of Method B [conversion of the hydroxyl group into a leaving group] can be carried out according to a method such as the one described above, and as shown in Reaction Scheme 10.

Step b) of Method B [reaction of the compound of general formula (II) with a compound of general formula (III)] can be carried out in the presence of a base in a reaction medium comprising an organic solvent, to obtain a compound of general formula (I), as described above in relation to Method A and shown in Reaction Scheme 11.

Likewise, step c) of Method B [conversion of a compound of general formula (I) into another compound of general formula (I)] can be carried out by conventional methods, as described above in relation to Method A.

In a particular embodiment, the transformation of said hydroxyl group into a leaving group defined in step a) of Method B is carried out by treatment with p-toluenesulfonyl chloride and step b) of said Method B is carried out by treatment with diphenylacetonitrile in the presence of LiNH₂ in a mixture of toluene and DMF.

In a particular embodiment, the compound of formula (I) is a compound in which the dotted line is absent, whereas in another particular embodiment, the obtained compound of formula (I) is a compound in which the dotted line represents a bond between the carbon atoms bound by said dotted line. Illustrative, non-limiting examples, of compounds of general formula (I) include 3-(1-carbamoyl-1,1-diphenylmethyl)-1-[2-(2,3-dihydrobenzofuran-5-yl)ethyl] pyrrolidine, 3(S)-(1-carbamoyl-1,1-diphenylmethyl)-1-[2-(2,3-dihydrobenzofuran-5-yl)ethyl] pyrrolidine, 3(R)-(1-carbamoyl-1,1-diphenylmethyl)-1-[2-(2,3-dihydrobenzofuran-5-yl)ethyl] pyrrolidine, solvates and pharmaceutically acceptable salts thereof. In a particular preferred embodiment, the compound of general formula (I) is 3-(S)-(-)-(1-carbamoyl-1,1-diphenylmethyl)-1-[2-(2,3-dihydrobenzofuran-5-yl)ethyl]pyrrolidine (Darifenacin), a solvate or a pharmaceutically acceptable salt thereof.

The following examples illustrate the invention and must not be interpreted in a limiting manner.

### EXAMPLE 1

### Obtaining 3-(S)-hydroxy-1-[2-(2,3-dihydrobenzofuran-5-yl)ethyl]-pyrrolidin-2,5-dione (S-Va)

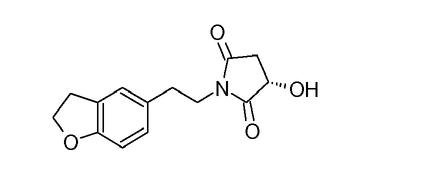

A mixture of 7.1 g (0.0437 moles) of 2-(2,3-dihydrobenzofuran-5-yl)-ethylamine and 5.9 g (0.0437 moles) of L-(-)-malic acid in 71.2 ml of xylene was heated at reflux for 3 hours. It was left to cool at room temperature and the solvent was concentrated at reduced pressure, exchanging it for EtOH up to a total volume of 35.6 ml. The resulting suspension was maintained under stirring at 0°C for 45 minutes, was filtered and washed with 7 ml of cold EtOH. 8 g (70% yield) of a white solid were obtained, having the following spectroscopic properties:
[α]²²₅₉₈= -61.6° (c 1, DCM)
[α]²²₃₆₅= -224.0° (c 1, DCM)
¹H-NMR (400 MHz, CDCl₃) : 2.58 (2H,dd, J₁=8Hz, J₂=16Hz), 2.79 (2H, t, J=8Hz), 2.94 (m, 2H), 3.13 (2H, t, J=8Hz), 3.66 (2H, t, J= 8Hz), 4.50 (2H, t, J=8Hz), 6.66 (1H, d, J=8Hz), 6.88 (1H, d, J=8Hz), 7.03 (1H, s) ppm.
¹³C-NMR (100 MHz, CDCl₃): 29.79 (CH2), 33.01 (CH2), 37.23 (CH2), 40.53 (CH2), 66.96 (CH), 71.35 (CH2), 109.24 (CH), 125.50 (CH), 127.51 (C), 128.45 (CH), 129.44(C), 159.07 (C), 174.05 (C), 178.30 (C) ppm.
LRMS (electrospray, positive ion): m/z [M⁺+H⁺] 262.1, [M⁺+Na⁺] 284.1

### EXAMPLE 2

### Obtaining 3-(S)-1-[2-(2,3-dihydrobenzofuran-5-yl)ethyl]-3-hydroxy-pyrrolidine (S-IVa)

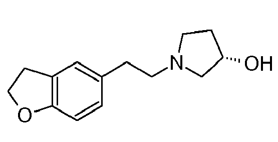

7.98 g (0.0306 moles) of 3-(S)-hydroxy-1-[2-(2,3-dihydrobenzofuran-5-yl)ethyl]-pyrrolidin-2,5-dione (S-Va) were added in portions to a suspension of 1.74 g (0.0459 moles) of LiAlH₄ in 26 ml of THF cooled at 5°C. The mixture was heated at 50°C for 6 hours. Once the reaction was finished, the mixture was cooled at 5-10°C, and 5.22 ml of THF/water=2/1, 1.74 ml of 20% NaOH aqueous solution w/v and 5.22 ml of water were added slowly and consecutively, considering the exotherm. The obtained suspension was left under stirring at room temperature for 30 minutes and the inorganic salts were filtered. The filtered liquid was distilled at reduced pressure until obtaining a residue, which was redissolved with 80 ml of dichloromethane (DCM). The organic phase was washed consecutively with 1N HCl aqueous solution (2x40 ml) and 20% NaOH aqueous solution w/v (2x40 ml). The organic extracts were concentrated at reduced pressure to give 6.77 g of the product in the form of a yellowish oil (95% yield) having the following spectroscopic properties:
[α]²²₅₉₈= -2.1° (c 1, DCM)
   ¹H-NMR (400 MHz, CH₃OD): 1.69 (1H, m), 2.06 (1H, m), 2.5 (2H, dd, J₁=4Hz, J₂=12Hz), 2.67 (6H, m), 2.83 (1H, dd, J₁=4Hz, J₂=12Hz), 3.08 (2H, t, J=8Hz), 4.32 (1H, m), 4.42 (2H, t, J= 8Hz), 6.59 (1H, d, J=8Hz), 6.87 (1H, d, J=8Hz), 7.02 (1H, s) ppm.
   ¹³C-NMR (100 MHz, CH₃OD): 30.72 (CH2), 35.25 (CH2), 35.53 (CH2), 53.90 (CH2), 60.01 (CH2), 63.68 (CH2), 71.49 (CH), 72.22 (CH2), 109.93 (CH), 126.27 (CH), 128.64(C), 129.07 (CH), 133.15 (C), 159.96 (C) ppm.
   LRMS (electrospray, positive ion): m/z [M⁺+H⁺] 262.1, [M⁺+Na⁺] 234.1

### EXAMPLE 3

### Obtaining 3-(S)-1-[2-(2,3-dihydrobenzofuran-5-yl)ethyl]-3-p-toluenesulfonyl pyrrolidine (S-IIa)

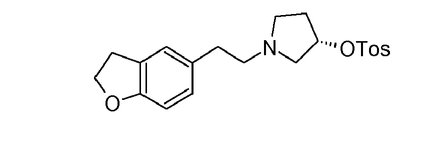

6.65 g (0.0349 moles) of p-toluenesulfonyl chloride (TsCl) were added in portions to a solution consisting of 6.77 g (0.029 moles) of 3-(S)-1-[2-(2,3-dihydrobenzofuran-5-yl)ethyl]-3-hydroxy-pyrrolidine (S-IVa), 6.1 ml (0.0436 moles) of Et₃N, 0.35 g (0.0029 moles) of DMAP and 67.7 ml of dichloromethane cooled at 10°C. The reaction mixture was maintained at 40°C for 5 hours. Once the reaction was finished, the reaction mixture was cooled at 15°C and 1 ml of water was added. The organic phase was washed with 1N HC1 aqueous solution (2x40 ml) and 7% NaHCO₃ aqueous solution (2x40 ml). The organic phase was concentrated at reduced pressure to obtain 10.3 g (91% yield) of the product in the form of a brown oil having the following spectroscopic properties:
[α]²²₃₆₅= -53.6° (c 1, DCM)
[α]²²₅₉₈= -12.2° (c 1, DCM)
¹H-NMR (400 MHz, CDCl₃) : 1.92 (1H, m), 2.09 (1H, m), 2.42 (3H, s), 2.47 (1H, m), 2.57-2.74 (7H, m), 3.11 (2H, t, J=8Hz), 4.47 (2H, t, J= 8Hz), 4.98 (1H, m), 6.64 (1H, d, J=8Hz), 6.85 (1H, d, J=8Hz), 6.98 (1H, s), 7.30 (2H, d, J=8Hz), 7.75 (2H, d, J=8Hz) ppm.
¹³C-NMR (100 MHz, CH₃OD) : 21.89 (CH3), 29.98 (CH2), 32.23 (CH2), 34.87 (CH2), 52.58 (CH2), 58.39 (CH2), 60.15 (CH2), 71.38 (CH2), 81.07 (CH).109.19 (CH), 125.32 (CH), 127.29 (C), 128.01 (CH), 130.08 (C), 130.09 (C), 134.15 (C), 144.99 ( C), 158.67 (C) ppm.
LRMS (electrospray, positive ion): m/z [M⁺+H⁺] 388.3

### EXAMPLE 4

### Obtaining 3-(S)-(-)-(1-carbonitrile-1,1-diphenylmethyl)-1-[2-(2,3-dihydrobenzofuran-5-yl)ethyl]pyrrolidine hydrobromide (S-Ia)

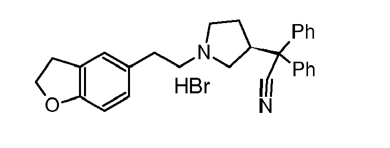

6.13 g (0.0317 moles) of diphenylacetonitrile were slowly added to a solution consisting of 10.3 g (0.0264 moles) of 3-(S)-1-[2-(2,3-dihydrobenzofuran-5-yl)ethyl]-3-*p-*toluenesulfonylpyrrolidine (S-IIa), 1.22 g (0.0529 moles) of NH₂Li, 20.5 ml of DMF and 102.5 ml of toluene heated at 110°C. The reaction mixture was maintained under stirring at said temperature (toluene reflux) for about 5 hours. Once the reaction was finished, the mixture was cooled at 15°C and 100 ml of water were added. After the separation of the phases, the organic phase was washed consecutively with 1N HCl aqueous solution (2x50 ml) and 20% NaOH aqueous solution (2x50 ml). The organic phase was concentrated to a residue at reduced pressure and was redissolved in 30 ml of acetone. Approximately 0.75 ml (0.013 moles) of 33% HBr in acetic acid were added little by little to this solution stirred at room temperature until reaching a pH of 3-4. The obtained suspension was maintained under stirring for 1 hour at room temperature and another hour at 0-5°C. The resulting solid was isolated by filtration to give 9.06 g (70% yield) of a white solid having the following spectroscopic properties:
[α]²⁵₃₆₅= 64° (c 0.1, MeOH) free base
[α]²⁵₃₆₅= -21° (c 0.1, MeOH) hydrobromide
   ¹H-NMR (400 MHz, CDCl₃): 1.81 (1H, m), 2.04 (1H, m), 2.44-2.57 (2H, m), 2.68 (4H, m), 2.75 (1H, dd, J₁=8Hz, J₂=16Hz), 2.99 (1H, dt, J₁=8Hz, J₂=12Hz), 3.12 (1H, t, J=8Hz), 3.42 (1H, m), 4.49 (1H, t, J=8Hz), 6.66 (1H, d, J=8Hz), 6.88 (1H, d, J=8Hz), 7.00 (1H, s), 7.24-7.45 (10H, m) ppm.
   ¹³C-NMR (100 MHz, CDCl₃): 28.65 (CH2), 29.88 (CH2), 34.89(CH2), 44.85 (CH), 54.08 (CH2), 56.93 (C), 57.97 (CH2), 58.41 (CH2), 71.23 (C).109.06 (CH), 121.65 (C), 125.18 (CH), 127.29 (C), 126.56 (CH), 127.06 (CH), 127.90 (CH), 129.02 (CH), 132.22 (C), 139.92 (C), 140.11 (C), 158.51 (C) ppm.
   LRMS (electrospray, positive ion): m/z [M⁺+H⁺] 409.4

### EXAMPLE 5

### Obtaining Darifenacin Hydrobromide

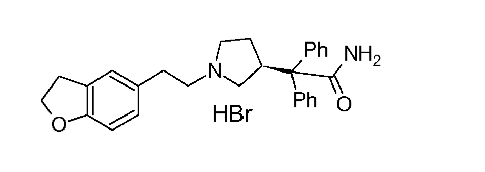

A suspension of 20.7 g (0.37 moles) of KOH in 90 ml of 2-methyl-2-butanol was heated at 60°C for 1 hour, then 9.06 g (0.0185 moles) of 3-(S)-(-)-(1-carbonitrile-1,1-diphenylmethyl)-1-[2-(2,3-dihydrobenzofuran-5-yl)ethyl]-pyrrolidine hydrobromide (S-Ia) were added; the resulting mixture was heated until reaching 108°C and was maintained at said temperature for 30 hours. Once the reaction was finished, the reaction mixture was cooled at room temperature and 90 ml of water were added. After the separation of the phases, the organic phase was concentrated to a residue at reduced pressure and was redissolved with 45 ml of acetone. Approximately 1.07 ml (0.0185 moles) of 33% HBr in acetic acid was added little by little to this solution stirred at room temperature, it was stirred at room temperature for 1 hour and the solvent was removed at reduced pressure. The resulting residue was redissolved with 100 ml of isopropanol and was maintained under stirring for 1 hour at room temperature and for half an hour at 0-5°C, giving a suspension. The resulting solid was isolated by filtration to give 6.77g (72% yield) of a white solid which coincides with commercial Darifenacin both in relation to its physical characteristics and by means of spectroscopic identification.

### EXAMPLE 6

### Obtaining 2-(2,3-dihydrobenzofuran-5-yl)-acetamide

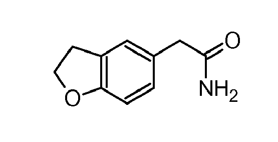

5 ml of DMF and a solution of 45 ml (0.617 moles) of thionyl chloride in 100 ml of acetonitrile were added consecutively to a solution of 100 g (0.560 moles) of (2,3-dihydrobenzofuran-5-yl)-acetic acid in 900 ml of acetonitrile. The reaction mixture was maintained at 45°C for 4 hours. Once the reaction was finished, the obtained solution was cooled at 15°C and was slowly added during approximately one hour to a mixture consisting of 800 ml of 25% NH₃ aqueous solution and 400 ml of acetonitrile cooled at 5°C. Once the reaction mixture was added, it was maintained at room temperature for 4 hours. Then 800 ml of water were added and it was stirred for 30 minutes; the obtained phases were separated and the aqueous phase was extracted with 2x 100 ml of acetonitrile. The combined extracts were concentrated under vacuum to a final volume of 500 ml. The obtained suspension was maintained under stirring at 5°C for 30 minutes and the solid was isolated by filtration, obtaining 96.5 g (97% yield) of a white solid having the following spectroscopic properties:
¹H NMR (400 MHz, DMSO): 3.09 (2H, t, J=8Hz), 3.26 (2H, s), 4.45 (2H, t, J=8Hz), 6.64 (1H, d, J=8Hz), 6.81 (1H, s), 6.93 (1H, d, J=8Hz), 7.09 (1H, s), 7.34 (1H, s) ppm.
   ¹³C NMR (100 MHz, DMSO): 29.12 (CH2), 41.69 (CH2), 70.79 (CH2), 108.39 (CH), 125.7 (CH), 127.09 (C), 128.21 (C), 128.33 (CH), 158.30 (C), 172.89 (C) ppm.
   LRMS (electrospray, positive ion): m/z [M⁺+Na⁺] 199.8

### EXAMPLE 7

### Obtaining 2-(2,3-dihydrobenzofuran-5-yl)-ethylamine (VIa)

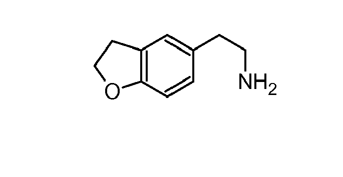

270 ml of BF₃-Et₂O were added slowly to a suspension consisting of 96.5 g (0.545 moles) of 2-(2,3-dihydrobenzofuran-5-yl)-acetamide, 41.3 g (1.09 moles) of NaBH₄ and 965 ml of THF at 5°C and under inert nitrogen atmosphere without the temperature rising. The mixture was progressively heated to an internal temperature of 55°C and was maintained at that temperature until the end of the reaction (about 7 hours). The reaction mixture was cooled at 0-5°C and 145 ml of water in 440 ml of THF were added without the temperature rising and maintaining nitrogen stream. The reaction mixture was left stirring for half an hour to an hour at room temperature and was cooled again at 5°C to add 580 ml of 20% NaOH aqueous solution. It was heated progressively to an internal temperature of 40°C and was stirred for 4 hours at that temperature. The mixture was left to cool and decanted and the organic phase was distilled at reduced pressure, obtaining 96.2 g (80 % yield) of 2-(2,3-dihydrobenzofuran-5-yl)-ethylamine.
¹H NMR (400 MHz, DMSO): 2.73 (2H, t, J=8Hz), 2.90 (2H, m), 3.08 (2H, t, J=8Hz), 4.43 (2H, t, J=8Hz), 6.64 (1H, d, J=8Hz), 6.90 (1H, d, J=8Hz), 7.08 (1H, s), 7.90 (2H, s) ppm.
¹³C NMR (100 MHz, DMSO): 29.28 (CH2), 32.63 (CH2), 40.61 (CH2), 71.06 (CH2), 109.03 (CH), 125.6 (CH), 127.85 (C), 128.25 (CH), 129.13 (C), 158.76 (C) ppm.
LRMS (electrospray, positive ion): m/z [M⁺+H⁺] 164.0, [M⁺-NH₂] 146.9

## Claims

1. A method for obtaining a compound of general formula (I) 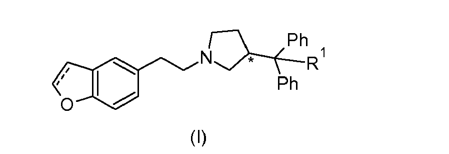 wherein
the dotted line is absent or represents a bond between the carbon atoms bound by said dotted line;
* represents an asymmetric carbon;
Ph represents a phenyl group; and
R¹ is CN, CONR²R³ or CO₂R², wherein R² and R³ represent independently of one another H, C₁-C₅ alkyl, or benzyl,
or an enantiomer, a solvate or a pharmaceutically acceptable salt thereof,
comprising:
a) transforming the hydroxyl group bound to the carbon in position 3 of the pyrrolidine present in a compound of general formula (IV) 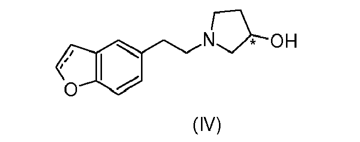 wherein the dotted line and the asterisk (*) have the previously mentioned meanings,
or an enantiomer, a solvate or a salt thereof,
into a leaving group to obtain a compound of general formula (II) 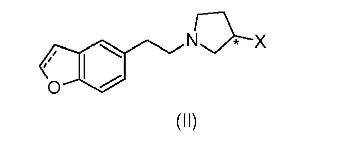 wherein the dotted line and the asterisk (*) have the previously mentioned meanings, and
X is a leaving group;
or an enantiomer, a solvate or a salt thereof,
b) reacting said compound of general formula (II), an enantiomer, a solvate or a salt thereof, with a compound of general formula (III), 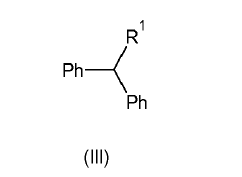 wherein Ph and R¹ have the previously mentioned meanings,
to obtain a compound of general formula (I), and, if desired,
c) converting the compound of general formula (I), an enantiomer, a solvate or a salt thereof, into another compound of general formula (I).

2. The method according to claim 1, wherein the transformation of the hydroxyl group into a leaving group defined in step a) is carried out by reacting said compound of general formula (IV) with a sulfonylating agent or with a halogenating agent to obtain a compound of general formula (II), wherein X is (C₁-C₄)alkylsulfonyloxy, arylsulfonyloxy, trifluoromethanesulfonyloxy or halide.

3. The method according to claim 2, wherein said sulfonylating agent is p-toluenesulfonyl chloride.

4. The method according to any of the previous claims, wherein the reaction of the compound of general formula (II) with a compound of general formula (III) in step b) is carried out in the presence of a base.

5. The method according to any of the previous claims, wherein the compound of formula (I) obtained is selected from 3-(1-carbamoyl-1,1-diphenylmethyl)-1-[2-(2,3-dihydrobenzofuran-5-yl)ethyl]pyrrolidine, 3-(S)-(1-carbamoyl-1,1-di-phenylmethyl)-1-[2-(2,3-dihydrobenzofuran-5-yl)ethyl]pyrrolidine, 3-(R)-(1-carbamoyl-1,1-diphenyl-methyl)-1-[2-(2,3-dihydrobenzofuran-5-yl)ethyl]pyrrolidine, enantiomers, solvates or pharmaceutically acceptable salts thereof; preferably 3-(S)-(-)-(1-carbamoyl-1,1-diphenyl-methyl)-1-[2-(2,3-dihydrobenzofuran-5-yl)ethyl]pyrrolidine (Darifenacin), or a solvate or a pharmaceutically acceptable salt thereof.

6. The method according to any of the previous claims, wherein said compound of general formula (IV), or an enantiomer, solvate or salt thereof, is obtained by means of reacting a compound of general formula (V) 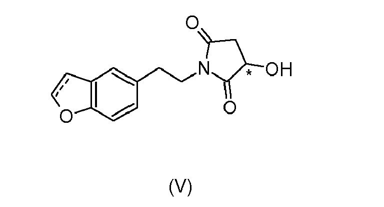 wherein
the dotted line is absent or represents a bond between the carbon atoms bound by said dotted line; and
* represents an asymmetric carbon;
or an enantiomer, a solvate or a salt thereof,
with a reducing agent.

7. The method according to claim 6, wherein said compound of general formula (V), or an enantiomer, a solvate or a salt thereof, is obtained by reacting a compound of general formula (VI) 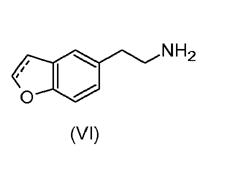 wherein
the dotted line is absent or represents a bond between the carbon atoms bound by said dotted line;
with an acid of formula (VII) 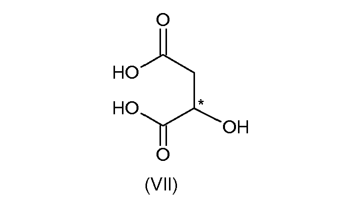 wherein
* represents an asymmetric carbon.

8. A compound selected from:
1-[2-(benzofuran-5-yl)ethyl]-3-*p*-toluenesulfonylpyrrolidine,
1-[2-(benzofuran-5-yl)ethyl]-3-methylsulfonyl-pyrrolidine,
1-[2-(benzofuran-5-yl)ethyl]-3-trifluoromethylsulfonyl-pyrrolidine,
1-[2-(benzofuran-5-yl)ethyl]-3-chloro-pyrrolidine, 1-[2-(2,3-dihydrobenzofuran-5-yl)ethyl]-3-*p-*toluenesulfonyl-pyrrolidine,
1-[2-(2,3-dihydrobenzofuran-5-yl)ethyl]-3-methylsulfonyl-pyrrolidine,
1-[2-(2,3-dihydrobenzofuran-5-yl)ethyl]-3-trifluoromethylsulfonyl-pyrrolidine,
1-[2-(2,3-dihydrobenzofuran-5-yl)ethyl]-3-chloropyrrolidine,
1-[2-(2,3-dihydrobenzofuran-5-yl)ethyl]-3-hydroxypyrrolidine,
1-[2-(benzofuran-5-yl)ethyl]-3-hydroxy-pyrrolidine, 3-hydroxy-1-[2-(2,3-dihydrobenzofuran-5-yl)ethyl]-pyrrolidin-2,5-dione,
3-hydroxy-1-[2-(benzofuran-5-yl)ethyl]-pyrrolidin-2,5-dione, and
enantiomers, solvates, or salts thereof.

9. The compound according to claim 8, selected from:
3-(S)-1-[2-(2,3-dihydrobenzofuran-5-yl)ethyl]-3-*p-*toluenesulfonyl-pyrrolidine,
3-(S)-1-[2-(benzofuran-5-yl)ethyl]-3-*p*-toluenesulfonylpyrrolidine,
3-(S)-1-[2-(2,3-dihydrobenzo-furan-5-yl)ethyl]-3-hydroxypyrrolidine,
3-(S)-1-[2-(benzofuran-5-yl)ethyl]-3-hydroxy-pyrrolidine, 3-(S)-hydroxy-1-[2-(2,3-dihydrobenzofuran-5-yl)ethyl]-pyrrolidin-2,5-dione,
3-(S)-hydroxy-1-[2-(benzofuran-5-yl)ethyl]-pyrrolidin-2,5-dione,
solvates or salts thereof.

10. A method for obtaining a compound of general formula (IV) 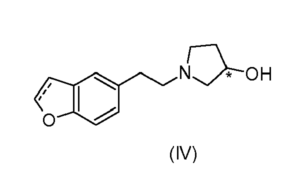 wherein
the dotted line is absent or represents a bond between the carbon atoms bound by said dotted line; and
* represents an asymmetric carbon;
an enantiomer, a solvate or a salt thereof,
comprising reacting a compound of general formula (V) 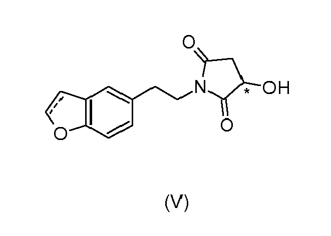 wherein the dotted line and the asterisk (*) have the previously mentioned meanings,
with a reducing agent.

11. The method according to claim 13, wherein said reducing agent is selected from the group consisting of lithium aluminum hydride (LiAlH₄), sodium bis(2-methoxyethoxy)aluminum hydride (REDAL), aluminum hydride (AlH₃), diborane, and mixtures thereof.
